# EUROPEAN PATENT SPECIFICATION

(11) **EP 4 559 413 B1**
(45) Date of publication and mention of the grant of the patent: **01.07.2026**
(21) Application number: 25169920.3
(22) Date of filing: 20.12.2021
(51) Int. Cl.: A61B 17/80, A61B 17/88

(54) **SURGICAL TOOLS AND SURGICAL TOOL SYSTEMS**
CHIRURGISCHE WERKZEUGE UND CHIRURGISCHE WERKZEUGSYSTEME
OUTILS CHIRURGICAUX ET SYSTÈMES D'OUTILS CHIRURGICAUX

(43) Date of publication of application: 28.05.2025
(62) Divisional of application: 21215868.7
(73) Proprietor: Stryker European Operations Limited, Carrigtwohill, Co. Cork T45 HX08 (IE)
(72) Inventor: KNÖPFLE, Christian, 78199 Bräunlingen (DE); KRAFT, Viktor, 79227 Schallstadt-Mengen (DE); GREINER, Karl, 78570 Mühlheim (DE); RETTICH, Jürgen, 78570 Mühlheim (DE); SCHMUCK, Manfred, 78570 Mühlheim (DE); KÖRNER, Uwe, 88637 Buchheim (DE)
(74) Representative: Röthinger, Rainer

(56) References cited:
- EP-A1- 3 626 192
- WO-A1-2014/055081
- US-A1- 2017 042 597
- US-A1- 2017 189 086
- US-A1- 2020 093 520

## Description

### Technical Field

The present disclosure generally relates to a plate bender and a plate bender system for surgical use.

### Background

In order to ensure that a bone plate lies flat on bone pieces to be connected and that the bone plate also extends in parallel to a potentially curved midline of these bone pieces, a plate bender can be used prior to or during surgery to manually bend the bone plate to properly fit the patient's bone geometry.

DE 102 24 005 A1 discloses a plate bender comprising a head for engaging the bone plate and a handle for being gripped by a hand. For bending a bone plate, two such plate benders are gripped with the left hand and the right hand, respectively. It has been found that depending on the distance between the bone plate engaging heads of the plate bender, the bending operation can be ergonomically cumbersome.

It has further been observed that while a bone plate is being fastened to a fractured bone piece, the bone plate tends to easily slip off the midline of the bone pieces and change its position. In some cases, the fracture may even be dislocated in such situations, which may require a repeated reduction of the fracture gap between adjacent bone pieces and may result in quite complicated re-adjustments, including in some cases a dismounting of a partially attached bone plate from a bone piece.

WO 2014/055081 A1 and EP 3 626 192 A1 describe a plate bender. Further background art can be found in US 2017/042597 A1, US 2017/189086 A1 and US 2020/093520 A1. EP 3 626 192 A1 describes a plate bender in accordance with the preamble of claim 1.

### Summary

The present invention is defined in claim 1 while preferred embodiments are set forth in the dependent claims.

The problem to be solved is to provide a convenient and versatile plate bender and plate bender system.

Solutions to the problem are provided in claims 1, 13 and 14.

A first embodiment of the present invention relates to a plate bender for bending a bone plate. The plate bender comprises a plate engagement portion and a handle portion coupled to the plate engagement portion. The handle portion is coupled to the plate engagement portion by a pivoting mechanism configured to change an angular orientation between the handle portion and the plate engagement portion.

A single plate bender may be configured to be operated completely one-handedly, which includes the operation of the plate engagement portion and the operation of the pivoting mechanism. Optionally, the operation can be such that the thumb of the hand gripping the handle portion can perform each operation while the hand remains tightly gripping the handle portion, for example by a pushing movement of the thumb.

The plate engagement portion has a longitudinal extension defining a first direction.

The handle portion is pivotable around the plate engagement portion in a plane angled to the first direction. The plane may be orthogonal to the first direction.

The plate bender may be configured to maintain individual ones of a plurality of angular orientations between the handle portion and the plate engagement portion. The angular orientations between the handle portion and the plate engagement portion may be discrete.

The plate bender may comprise a locking member displaceable between a locking position, in which a particular angular orientation is maintained, and an unlocking position, in which the handle portion can pivot around the plate engagement portion to change the angular orientation. The locking member may be provided at a region of the plate bender closer to the plate engagement portion than to a free longitudinal end of the handle portion. The locking member may be configured to engage a particular one of multiple locking portions to maintain a particular angular orientation. The locking member may be provided at the plate engagement portion and the locking portions may be provided on the handle portion, or vice versa.

The locking member may be spring-biased to assume the locking position and configured to be manually displaced against the spring-bias to assume the unlocking position. The locking member may be configured to be manually pushed towards the handle portion and thereby displaced to the unlocking position, and to return to the locking position when being released. The locking member may be configured to be manually pushed towards the handle portion in a second direction, which is either identical or parallel to the first direction.

The plate engagement portion comprises a displacement member displaceable between a plate engagement position, in which a plate engagement is maintained, and a plate disengagement position, in which a plate disengagement is provided. The displacement member may be provided at a region of the plate bender closer to the plate engagement portion than to a free longitudinal end of the handle portion.

Another example of the present disclosure, which example is not according to the presently claimed invention but useful for understanding the invention, relates to a plate bender comprising a plate engagement portion and a handle portion coupled to the plate engagement portion. The plate engagement portion comprises a displacement member, which can be displaced between a plate engagement position, in which a plate engagement is maintained, and a plate disengagement position, in which a plate disengagement is provided.

The displacement member may be spring-biased to assume the plate engagement position and configured to be manually displaced against the spring-bias to the plate disengagement position. The displacement member may be configured to be manually pushed towards the plate engagement portion to assume the plate disengagement position, and to return to the plate engagement position when being released. The plate engagement portion may comprise a through-hole configured to act as a drill or screw guide.

Another embodiment of the present invention relates to a plate bender system comprising a first and a second plate bender as described herein for left-hand and right-hand use, respectively. Each of the two plate benders may have a specific ergonomic form adapted to left-hand use and right-hand use, respectively. Alternatively, the two plate benders may have the same ergonomic form, so that the user can freely choose which plate bender to grip with which hand.

Another embodiment of the present invention relates to a plate bender system comprising at least one plate bender as described herein comprising a through-hole configured to act as a drill or screw guide and at least one bone plate having a plate opening. The plate engagement portion may be configured to align the plate opening, when the plate is engaged, with the through-hole configured to act as the drill or screw guide.

A further example of the present disclosure, which example is not according to the presently claimed invention but useful for understanding the invention, relates to plate holding forceps for one-hand use, wherein the forceps are configured to press a bone plate to bone. The forceps comprise a clamp portion, including a first jaw and second jaw movable towards and away from each other. The first jaw includes a through-bore configured to be aligned with a hole of a bone plate engaged by the plate holding forceps. The forceps further comprise an adjustment portion configured to adjust a distance between the jaws. The adjustment portion includes a first handle part and a second handle part manually movable with the fingers of one hand relative to each other and configured to thereby adjust the distance between the jaws.

The relative movement distance between the first handle part and the second handle part may correspond, e.g., linearly, to the relative movement distance between the jaws. The movement of the handle parts toward each other may cause a movement of the jaws towards each other, while a movement of the handle parts away from each other may cause a movement of the jaws away from each other. For this purpose, the first handle part may be fixedly connected to the first jaw and the second handle part may be fixedly connected to the second jaw. The first handle part and the second handle part may be two longitudinal parts slidably connected by their lateral sides, e.g., only by their lateral sides.

The plate holding forceps may further comprise a ratchet mechanism. The ratchet mechanism may have an active state, in which the jaws can be moved towards but not away from each other, and an inactive state, in which the jaws can be moved at least away from each other (e.g., towards and away from each other).

A still further example of the present disclosure, which example is not according to the presently claimed invention but useful for understanding the invention, relates to a plate holding forceps for one-hand use, wherein the forceps are configured to press a bone plate to bone. The forceps comprise a clamp portion, including a first jaw and second jaw movable towards and away from each other, wherein at least the first jaw includes a through-bore configured to be aligned with a hole of a bone plate engaged by the plate holding forceps. The forceps further comprise an adjustment portion configured to adjust a distance between the jaws, wherein the adjustment portion includes a ratchet mechanism having an active state, in which the jaws can be moved towards but not away from each other, and an inactive state, in which the jaws can be moved at least away from each other (e.g., towards and away from each other). The through-bore may comprise an inner funnel shape.

The ratchet mechanism may be located closer to the adjustment portion than to the clamp portion. Additionally, or alternatively, the ratchet mechanisms may be configured to be manually operated with the same hand that operates the adjustment portion.

In particular, the adjustment portion and the ratchet mechanism may both be operated by a pushing and/or pulling movement. Each operation may in some implementations be performed along a different straight line, for example along straight lines which are orthogonal to each other. The ratchet mechanism may comprise a manually operable switch configured to move the ratchet mechanism into the inactive state. The switch may be located on the first handle part or on the second handle part.

The plate holding forceps may further comprise a variable visual indication indicating a parameter that depends on the actual distance between the jaws. This parameter may be a number corresponding to the screw length to be used for fastening the bone plate to the bone piece.

The adjustment portion may be syringe-shaped with a plunger part (which includes the first handle part) and a barrel part (which includes the second handle part). The plunger part may be guided inside the barrel part and axially protrude from the barrel part on one side thereof. The above-mentioned visual indication may be provided on the plunger part. It may be etched or laser engraved into the plunger part.

The above-mentioned switch may be a rocker arm pivotably hinged to the barrel part to pivot around a barrel pivot axis laterally fixed to the barrel part. One end of the switch may be biased away from the barrel part, e.g., by means of a switch spring, such that the opposite end of the switch is automatically forced against the plunger part such that the ratchet mechanism assumes the active state. Moving the one end of the switch against its bias and towards the barrel part is configured to cause the ratchet mechanism to assume the inactive state.

The plunger part may include a plunger flange portion and the barrel part may include a barrel flange portion. The barrel flange portion optionally includes opposite lateral wings, which optionally are held in a rotatable manner on the barrel part and further optionally have a width in a plane parallel to the transverse cross-sectional plane of the barrel part less than the width of the largest cross-sectional profile of the barrel part. The plunger and barrel flange portions may remain within lateral boundaries of a circumferential section of a largest cross-sectional profile of the barrel part.

The first jaw may be laterally spaced apart from the handle parts by a distance defined by a lateral extension of a first step portion. The first step portion may have an upside-down L-shape with the short side end of the L-shape fixed to the plunger part and the longer side end fixed to the first jaw, both orthogonally, for example.

The second jaw may be laterally spaced apart from the handle parts by a corresponding second step portion, wherein both step portions may engage in a guiding manner. The second step portion may have an upside-down L-shape with the short side end of the L-shape fixed to the barrel part and the longer side end fixed to the second jaw, both orthogonally, for example.

The engagement in a guiding manner may be provided by means of a rail slot, e.g., at one of the first or second step portions, and a sliding member, e.g., at the other one of the first or second step portions, slidably held in the rail slot to allow a relative sliding movement in-between only in a direction, in which the jaws are moved towards and away from each other.

The plate holding forceps may further comprise a guiding protrusion. The guiding protrusion may protrude from the first jaw in a direction opposite to the second jaw. The through-bore may extend through the guiding protrusion.

The first jaw may comprise a U-shaped cross-section, which is open towards the second jaw. The cross-section may also be L-shaped with the base of the L-shape forming the free end of the first jaw. At least one of the jaws may comprise a corrugated surface or a pointed surface.

Further provided is a plate holding forceps system comprising at least one plate holding forceps as described herein and at least one bone plate configured to be clamped by the clamp portion.

### Brief Description of the Drawings

Further details and advantages of the present disclosure will become apparent from the following description taken in conjunction with the drawings, wherein:
- Fig. 1: shows a fractured bone, which is broken into two separate bone pieces;
- Fig. 2: shows the lined-up bone pieces of Fig. 1 with a bone plate on top;
- Fig. 3: shows a top view of two equally angled plate benders engaged to the same bone plate;
- Fig. 4: shows a top view of two differently angled plate benders engaged to the same bone plate;
- Fig. 5: shows a perspective view of a plate bender above a bone plate, prior to engagement;
- Fig. 6: shows a perspective view of the plate bender and bone plate of Fig. 5, during engagement;
- Fig. 7: shows a perspective view of the plate bender and bone plate of Fig. 6, after engagement;
- Fig. 8: shows a perspective view of the plate bender and bone plate of Fig. 7, after an angle change between the handle portion and the plate engagement portion;
- Fig. 9: shows a perspective view of the plate bender of Fig. 3 to 8 with a cross-section view of a part of the plate engagement portion and a part of the handle portion, wherein the pivoting mechanism in-between is in a locked state;
- Fig. 10: shows the perspective view of the plate bender of Fig. 9, wherein the pivoting mechanism is in an unlocked state;
- Fig. 11: shows a lateral cross-section view of the plate engagement portion in an initial plate engagement position - without a bone plate;
- Fig. 12: shows the lateral cross-section of Fig. 11 with the plate engagement portion in a plate disengagement position - with a bone plate;
- Fig. 13: shows the lateral cross-section of Fig. 11 with the plate engagement portion in a plate engagement position - with a bone plate;
- Fig. 14: shows a perspective view of a plate holding forceps for one-hand use pressing a bone plate to one of two bone pieces;
- Fig. 15: shows a perspective view of two of the plate holding forceps of Fig. and an enlargement of said view, wherein each plate holding forceps press the same bone plate at opposite ends thereof to a different one of the two bone pieces;
- Fig. 16: shows a side view of the plate holding forceps with the first jaw and the second jaw being distanced from each other;
- Fig. 17: shows a side view of the plate holding forceps with the first jaw and the second jaw being distanced less from each other than in Fig. 16 and tightly holding the bone plate to one of the two bone pieces;
- Fig. 18: shows a perspective view of the plate holding forceps from the bottom side thereof with the bone plate held by the first jaw;
- Fig. 19: shows a perspective view of the plate holding forceps from the top side thereof;
- Fig. 20: shows a perspective view of the plate holding forceps from the top side thereof with a screwdriver;
- Fig. 21: shows a cross-section of Fig. 20 in a state, wherein a screw is not fully countersunk; and
- Fig. 22: shows the cross-section of Fig. 21 in a state, wherein the screw is fully countersunk.

### Detailed Description

In the following description of examples, the same reference numerals are used to denote the same or similar components.

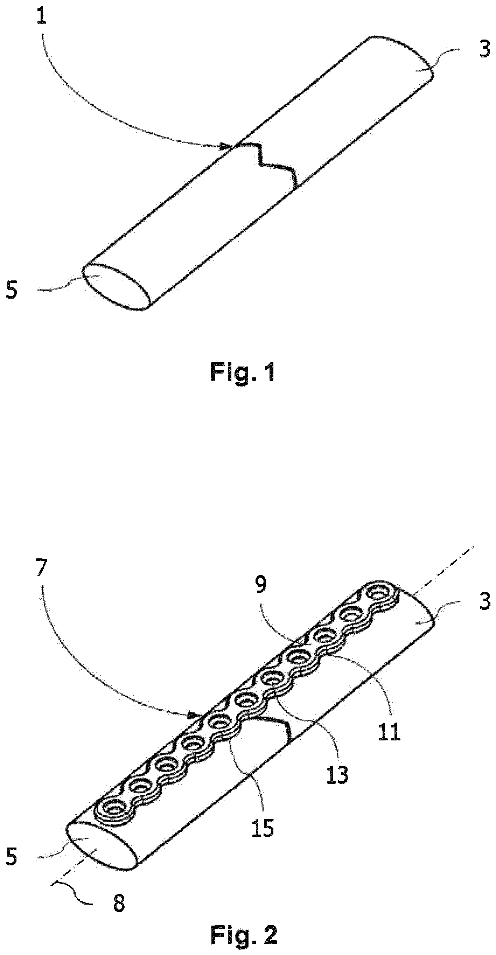

The present disclosure provides tools and tool systems for use in a surgical procedure in which a bone plate needs to be attached to bone. Fig. 1 shows a fractured bone 1, which is broken into two separate bone pieces 3, 5. The broken ends of the bone pieces 3, 5 line up to form the original bone 1. In some variants, the bone 1 may be a rib and the bone pieces 3, 5 may be rib pieces. In other variants, the fractured bone may be a mandible or other bone.

Fig. 2 shows the lined-up bone pieces 3, 5 of Fig. 1 with a bone plate 7 lying on top and extending in parallel to a longitudinal midline 8 of the bone pieces 3, 5. The bone plate 7 has a linear extension with a top surface 9 and a bottom, here bone-facing, surface 11 as well as multiple openings 13 for receiving bone screws (not shown) extending between the top surface 9 and the bottom surface 11. In this case, the openings 13 are through-holes. The openings 13 are arranged in a pattern, for example in a row. Of course, the pattern of the openings 13 may be chosen freely and, for example, only some of the openings 13 may be arranged in a row.

The openings 13 are equally spaced from each other, but could have a non-equal spacing. Each opening 13 is defined by a ring-shaped structure, which is connected to the ring-shaped structure of an adjacent opening 13 to form the single row of connected ring-shaped structures.

The bone plate 7 of Fig. 2 provides an engagement part 15 capable of being engaged, e.g., held, gripped or fixed, by one of the below described tools. The engagement part 15 is shown to be a lateral bulge that protrudes from both lateral sides of the bone plate 7 in a direction orthogonal to the axial extension of the bone plate (and of the openings 13). Of course, instead of being a protruding bulge, the engagement part 15 may have a different configuration. As an example, the engagement part 15 may be a lateral recess, e.g., groove, in the bone plate 7. While the engagement part 15 is shown as continuously surrounding the bone plate 7, it may alternatively be discontinuously surrounding the bone plate 7.

In order to ensure that the bone plate 7 lies flat on the bone pieces 3, 5 and also in parallel to the midline of the bone pieces 3, 5, the below described first tool, i.e., plate bender, can be used prior to and/or during surgery. For example, there may be a need for an in-plane and/or an out-of-plane bending of the bone plate 7.

In order to ensure that the bone plate 7 remains flat on the bone pieces 3, 5 and also in parallel to the midline of the bone pieces 3, 5 while avoiding a dislocation of the fracture, the below described second tool, i.e., plate holding forceps, can be used during surgery.

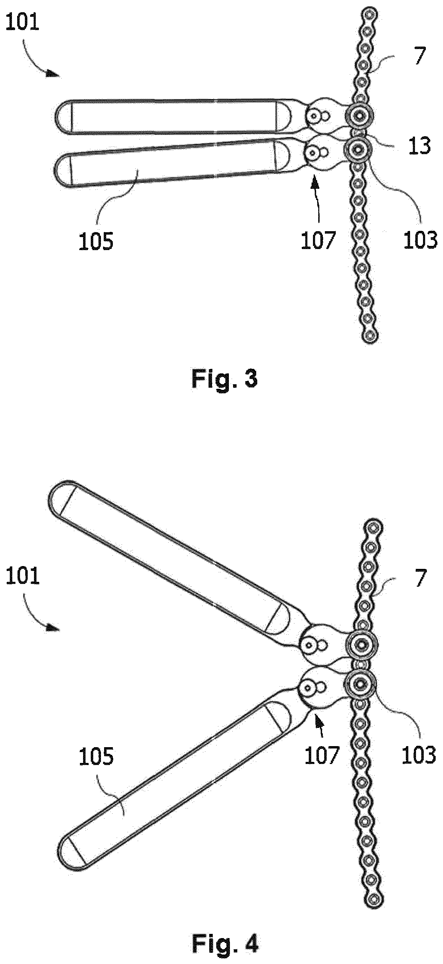

Fig. 3 shows a top view of two equally angled plate benders 101 engaged to the same bone plate 7. Each plate bender 101 comprises a plate engagement portion 103 and a longitudinal handle portion 105 coupled to the plate engagement portion 103. The handle portion 105 is coupled to the plate engagement portion 103 by a pivoting mechanism 107 configured to change an angular orientation between the handle portion 105 and the plate engagement portion 103.

As can be seen here, both plate benders 101 are engaged to the bone plate 7 in structurally close proximity to each other, i.e., with one opening 13 in-between. Further, the angle between the handle portion 105 and the plate engagement portion 103 of both plate bender's 101 is the same, i.e., the plate benders are equally angled. This results in the respective handle portions 105 being so close to each other that it becomes difficult to ergonomically, i.e., comfortably, grasp each of the respective handle portions 105 with one hand in order to bend the bone plate 7 by means of a relative movement between the respective handle portions 105.

Fig. 4 shows a top view of two differently angled plate benders 101 engaged to the same bone plate 7. The difference between Fig. 4 and Fig. 3 is that in Fig. 4 the angle between the handle portion 105 and the plate engagement portion 103 in both plate benders 101 differs. This results in the respective handle portions 105 being so distant from each other that it becomes easier and more comfortable to grasp each of the respective handle portions 105 with one hand in order to bend the bone plate 7 by means of a relative movement between the respective handle portions 105.

The ability to change the angular orientation between the handle portion 105 and the plate engagement portion 103 is provided by the pivoting mechanism 107. Since the plate engagement portion 103 engages the bone plate 7 fixedly, i.e., such that the bone plate 7 cannot escape from the engagement with the engagement portion 103, the plate bender 101 can be used to bend the bone plate 7.

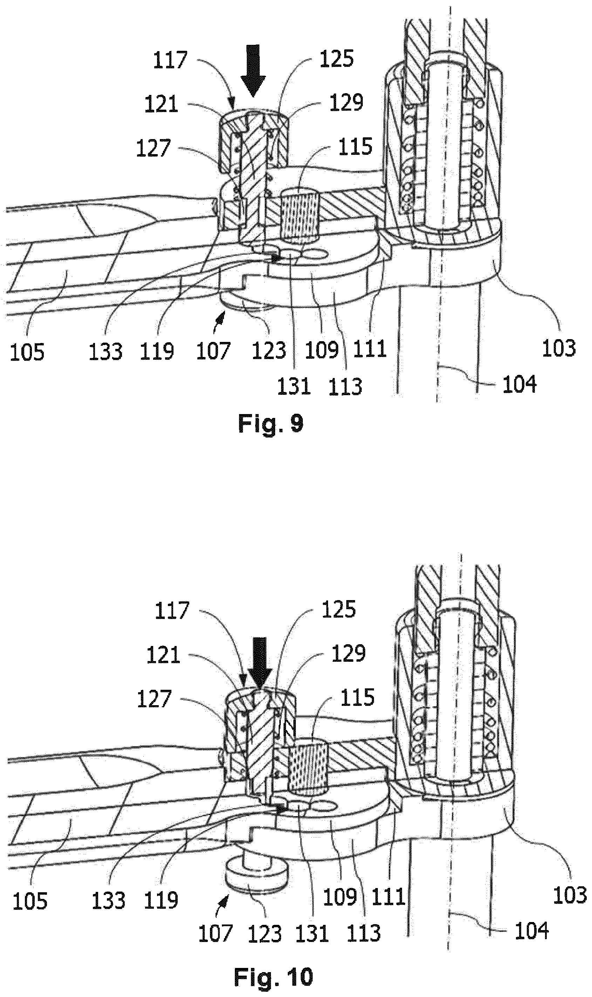

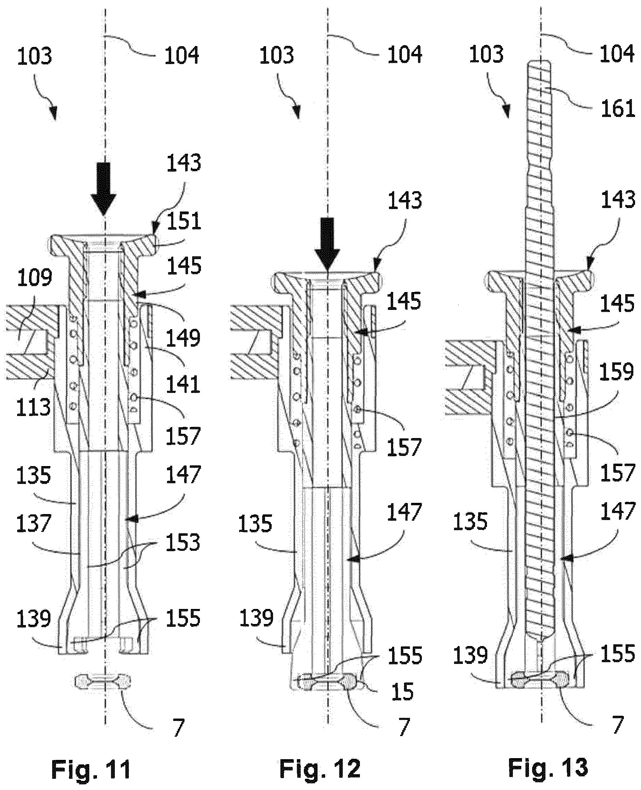

The pivoting mechanism 107 is described in detail below with reference to Figs. 9 and 10. The plate engagement portion 103 is described in detail below with reference to Figs. 11 to 13.

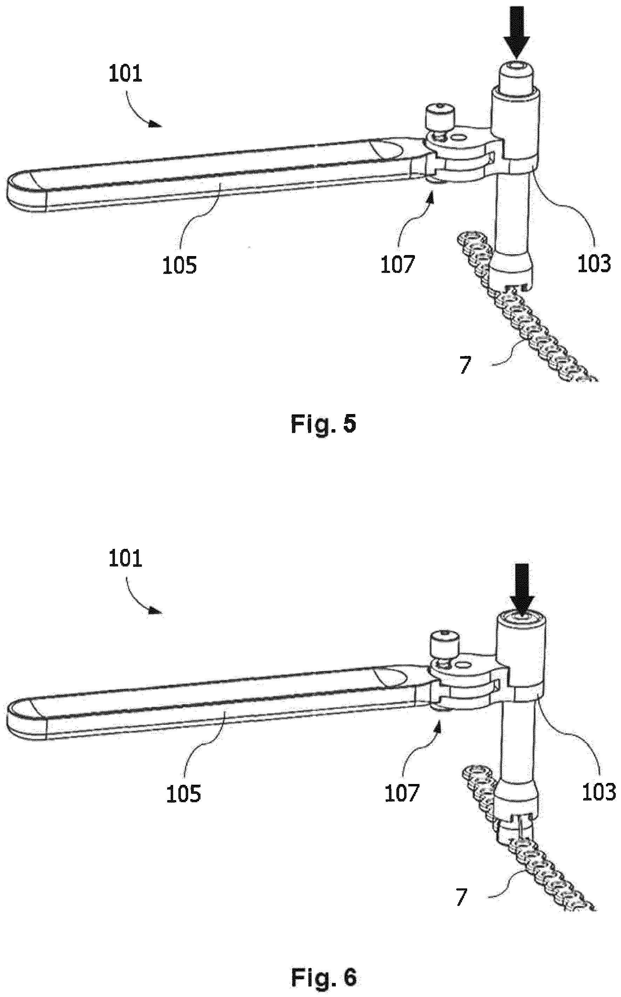

Figs. 5 to 8 show a perspective view of the procedure of engaging a plate bender 101 to a bone plate 7, and changing the angle between the handle portion 105 and the plate engagement portion 103 by means of the pivoting mechanism 107. While this procedure is shown in a specific order, i.e., engagement followed by change of angular orientation, the order can be reversed, i.e., change of angular orientation followed by engagement.

In these figures, as well as in the remaining figures, a bold arrow indicates a force about to be exercised on a structural part, when the tip of the arrow is still distant from the respective structural part, to which the arrow points. The same type of arrow indicates a force being exercised and/or maintained on a structural part, when the tip of the arrow touches the respective structural part.

Fig. 5 shows a perspective view of a plate bender 101 above a bone plate 7, prior to engagement. A black arrow pointing downwards indicates where and in which direction a force has yet to be exercised in order to initiate the engagement with the bone plate 7. The plate engagement portion 103 and the handle portion 105 have an initial angular orientation between each other.

Fig. 6 shows a perspective view of the plate bender 101 and bone plate 7 of Fig. 5, during engagement. A black arrow pointing downwards indicates that the force already indicated in Fig. 5 is now being exercised and maintained in order to proceed with the engagement procedure. Then, once the force is no longer maintained, the engagement procedure is completed. All the while, the plate engagement portion 103 and the handle portion 105 maintain the initial angular orientation between each other.

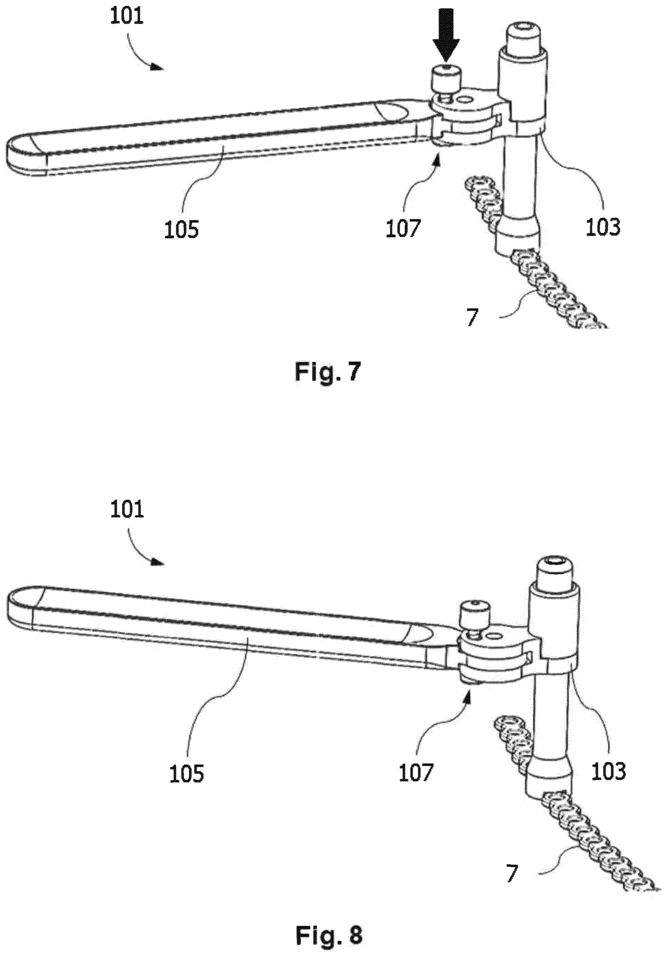

Fig. 7 shows a perspective view of the plate bender 101 and bone plate 7 of Fig. 6, after engagement. A black arrow pointing downwards indicates where and in which direction a force has yet to be exercised in order to initiate the change in angular orientation between the plate engagement portion 103 and the handle portion 105 from the initial angular orientation between each other.

Fig. 8 shows a perspective view of the plate bender 101 and bone plate 7 of Fig. 7, after an angle change between the handle portion 105 and the plate engagement portion 103. The new angular orientation differs from the initial angular orientation between the plate engagement portion 103 and the handle portion 105 and is maintained by the pivoting mechanism 107.

The plate engagement procedure performed in Figs. 5 and 6 is explained in detail below with reference to Fig. 11 to 13. The angle change procedure performed in Figs. 7 and 8 is explained in detail below with reference to Figs. 9 and 10. Of course, the order in which each of these procedures is performed is arbitrary.

The plate bender 101 is configured to be operated completely one-handed, which includes the operation of the plate engagement portion 103 and/or the operation of the pivoting mechanism 107. Here, both operations are performed by manually displacing, e.g. pushing, a member, e.g., a button, at a region of the plate bender 101, which is closer to the plate engagement portion 103 than to a free longitudinal end of the handle portion 105, e.g., at the plate engagement portion 103 of the plate bender 101. In summary, the thumb of the hand gripping the handle portion 105 can perform each operation while the hand remains tightly gripping the handle portion 105.

Fig. 9 shows a perspective view of the plate bender 101 with a cross-section view of a part of the plate engagement portion 103 and a part of the handle portion 105, wherein the pivoting mechanism 107 provided in-between is in a locked state.

The pivoting mechanism 107 is formed by a circular plate-shaped handle end 109, which is inserted into a corresponding recess 111 of a laterally protruding mount 113 of the plate engagement portion 103. The handle end 109 is pivotably hinged to the plate engagement portion 103 within the recess 111 by means of a pivot axis 115 connecting the circular plate-shaped handle end 109 and the mount 113 pivotably.

Thereby, the handle portion 105 is pivotable around the plate engagement portion 103. Here, this pivotability is in a plane, in which the handle portion 105 extends longitudinally and which is orthogonal to a first axis 104, in which the plate engagement portion 103 extends longitudinally.

In order to maintain individual ones of a plurality of discrete angular orientations between the handle portion 105 and the plate engagement portion 103, a locking member 117 and locking portions 119 are provided.

The locking member 117 is provided at and through the entire mount 113 of the plate engagement portion 103 in parallel to the pivot axis 115. The locking portions 119 are provided at and through the handle end 109 of the handle portion 105. Alternatively, the locking member 117 may be provided at the handle end 109 of the handle portion 105 and the locking portions 119 may be provided at the mount 113 of the plate engagement portion 103, if the engagement portion 103 comprises the circular plate-shaped end and the handle portion 105 comprises the mount.

The locking member 117 comprises a cylindrical pin 121 with a coaxial flat-shaped head 123 on one end and a cup shaped button 125 at the other longitudinal end. In-between both ends the cylindrical pin 121 comprises a continuous ring-shaped pin recess 127. Also, both ends of the locking member 117 extend beyond the mount 113. The locking member 117 further comprises a helical locking spring 129, which surrounds the pin 121 and biases the cup shaped button 125 away from the mount 113.

The locking portions 119 comprise parallel intersecting cylindrical through-holes 131 equally distanced around the pivot axis 115 and from each other. The inner diameter of these through-holes 131 is equal to or larger than the general diameter of the pin 121. However, their intersections 133 define a passage with a radial distance, i.e. a distance in a direction orthogonally towards the pivot axis 115, that is smaller than the general diameter of the pin 121 but equal to or larger than the outer diameter of the pin 121 at the ring-shaped recess 127.

In the locked state of the pivoting mechanism, the locking member 117 is in a locking position, which is automatically maintained and returned to by means of the bias of the spring 129. In this state (or position), the pin 121 is located at least partially with a general diameter portion inside one of the through-holes 131 of the locking portions 119. Since this general diameter portion of the pin 121 cannot pass through the intersections 133, the handle portion 105 is prevented from pivoting around the plate engagement portion 103.

These locking portions 119 with their cylindrical through-holes 133 provide discrete angular orientations between the handle portion 105 and the plate engagement portion 103.

Fig. 10 shows the perspective view of the plate bender 101 of Fig. 9, wherein the pivoting mechanism 107 is in an unlocked state. In the unlocked state of the pivoting mechanism 107, the locking member 117 is in an unlocking position, which is manually and temporary maintained against the bias of the spring 129. Here, the button 125 is pushed against the biasing force of the spring 129. The spring 129 is compressed towards the mount 113 and the pin 121 is moved further into the mount 113. This can be seen here by the displacement of the head 123 and the button 125 of the pin 121, which have moved downward compared to their respective positions in Fig. 9. In this state/position, the pin 121 is located with only its part comprising the recess 127 inside one of the through-holes 131 of the locking portions 119. Since the outer diameter of the recess 127 is equal to or less than the passage of the intersections 133, the pin 121 can pass freely through the intersections 133, which allows the handle portion 105 to pivot around the plate engagement portion 103.

As soon as the button 125 is released, the pivoting mechanism 107 will return to its initial position, as shown in Fig. 9, as long as one of the through-holes 131 of the locking portions 119 is arranged to be coaxial to the pin 121.

Fig. 11 shows a lateral cross-section view of the plate engagement portion 103 in an initial plate engagement position - without contact to a bone plate 7. The plate engagement portion 103 comprises a longitudinal main body 135 with a through-opening 137 extending along the first axis 104. The bottom end 139 of the main body 135 is funnel-shaped, such that the through-opening 137 becomes wider towards the end of the bottom end 139 of the main body 135. The top end 141 of the main body 135, which is opposite to the bottom end 139, is where the mount 113 of the plate engagement portion 103 protrudes laterally from the main body 135 to form a U-shaped cross-section, in which the handle end 109 is inserted. Inside the main body 135, a displacement member 143 is provided, which is movable along the first axis 104 inside the through-opening 137 and relative to the main body 135. The displacement member 143 comprises an upper portion 145 and a lower portion 147.

The upper portion 145 comprises a cylinder 149 with a circumferential flange 151 at the top and protruding laterally from the cylinder 149. The flange 151 is configured to abut the main body 135 and prevent the displacement member 143 from being moved further into or entirely through the through-opening 137 in a top to bottom direction along the first axis 104.

The lower portion 147 comprises two arms 153, which extend from the bottom of the upper portion 145 symmetrically about the first axis 104, are distanced from each other in a direction orthogonal to the first axis 104, and each end with a hook-shaped engagement end 155, where the hooks face each other. Both engagement ends 155 form an outer shape that corresponds to the inside of the funnel-shaped bottom end 139. Thus, the engagement ends 155 are configured to engage the bottom end 139 in a form-fit and prevent the displacement member 143 from being moved further into or entirely through the through-opening 137 in a bottom to top direction along the first axis 104.

A helical engagement spring 157, which is arranged between the upper portion 145 and the main body 135, is configured to bias the displacement member 143 in a top direction along the first axis 104 in order to engage the engagement ends 155 to the bottom end 139 and distance the flange 151 from the main body 135.

In this state, the engagement ends 155 of the displacement member 143 are biased by the bottom end 139 towards each other and their distance in a direction orthogonal to the first axis 104 is reduced compared to a state, in which the bottom end 139 is absent or distant in the direction of the first axis 104. Thereby, the bone plate 7 shown in Fig. 11 cannot be engaged.

Fig. 12 shows the lateral cross-section view of Fig. 11 with the plate engagement portion 103 in a plate disengagement position - with contact to the bone plate 7. In order to engage the bone plate 7, the top of the upper portion 145 of the displacement member 143 has to be pushed towards the main body 135 of the displacement member 143 and against the biasing force of the engagement spring 157. This will cause the engagement ends 155 of the lower portion 147 of the displacement member 143 to be brought out of their form-fit with the bottom end 139 of the lower portion 147. Thereby, the engagement ends 155 are allowed to increase their distance from each other in the direction orthogonal to the first axis 104, whereby the bone plate 7 can be gripped around its engagement part 15 by means of the hook-shaped engagement ends 155.

Fig. 13 shows the lateral cross-section of Fig. 11 with the plate engagement portion 103 in a plate engagement position - with contact to the bone plate 7. As soon as the pushing force on the top of the upper portion 145 of the displacement member 143 is removed, the engagement spring 157 returns the displacement member 143 to its initial position, as shown in Fig. 11. The bone plate 7, which has already been gripped in Fig. 12, is now fixedly held by the engagement ends 155 of the lower portion 147 of the displacement member 143. This is due to the engagement ends 155 being biased towards each other in the direction orthogonal to the first axis 104 and prevented from increasing their distance from each other. Thereby, the bone plate 7 is securely trapped.

Here, the displacement member 143 comprises an engagement through-hole 159 extending along the first axis 104 and through the entire displacement member 143. The engagement through-hole 159 is cylindrical with a minimum diameter, such that it is configured to act as a drill or screw guide, which has a maximum outer diameter that is equal to or less than the minimum diameter of the engagement through-hole 159. As shown in Fig. 13, a drill 161 is partially inserted into the engagement through-hole 159 to exemplary illustrate the afore-mentioned optional provision of an engagement through-hole 159.

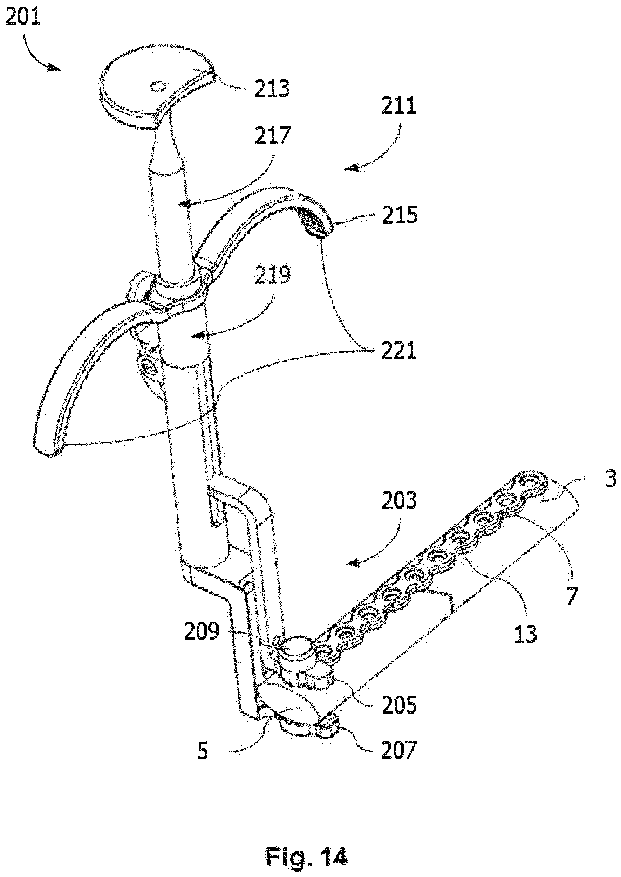

Fig. 14 shows a perspective view of a plate holding forceps 201 for one-hand use pressing a bone plate 7 to one 5 of two bone pieces 3, 5. A clamp portion 203 of the plate holding forceps 201, which clamp portion 203 includes a first jaw 205 and a second jaw 207 movable towards and away from each other, enables this pressing. The first jaw 205 includes a jaw through-bore 209 configured to be aligned with one of several hole-shaped openings 13 of the bone plate 7 engaged by the plate holding forceps 201.

An adjustment portion 211 of the plate holding forceps 201 is configured to adjust a distance between the first jaw 205 and the second jaw 207. For this purpose, the adjustment portion 211 includes a first handle part 213 and a second handle part 215 manually movable with the fingers of one hand relative to each other and configured to thereby adjust the distance between the first jaw 205 and the second jaw 207.

The adjustment portion 211 is in this case syringe-shaped, i.e., it comprises a plunger part 217, which includes the first handle part 213, and a barrel part 219, which includes the second handle part 215. The plunger part 217 includes a plunger flange portion as the first handle part 213 and the barrel part 219 includes a barrel flange portion as the second handle part 215, wherein the plunger flange portion and the barrel flange portion remain within lateral boundaries of a circumferential section of a largest cross-sectional profile of the barrel part 219. The barrel flange portion includes opposite lateral wings 221. While these wings 221 are fixedly held on or integral with the barrel part 219 they could form a separate part, which is held in a rotatable manner on the barrel part 219. Here, the wings 221 have a width in a plane parallel to the transverse cross-sectional plane of the barrel part 219 less than the width of the largest cross-sectional profile of the barrel part 219. Alternatively, the first handle part 213 and the second handle part 215 may be two longitudinal parts slidably connected by their lateral sides, e.g., only by their lateral side, such that no part is enclosed by the other in contrast to the plunger part 217 being enclosed by the barrel part 219. In this case, the plunger flange portion and the barrel flange portion can be provided in any shape even as respective first handle part flange and/or as second handle part flange, or omitted entirely.

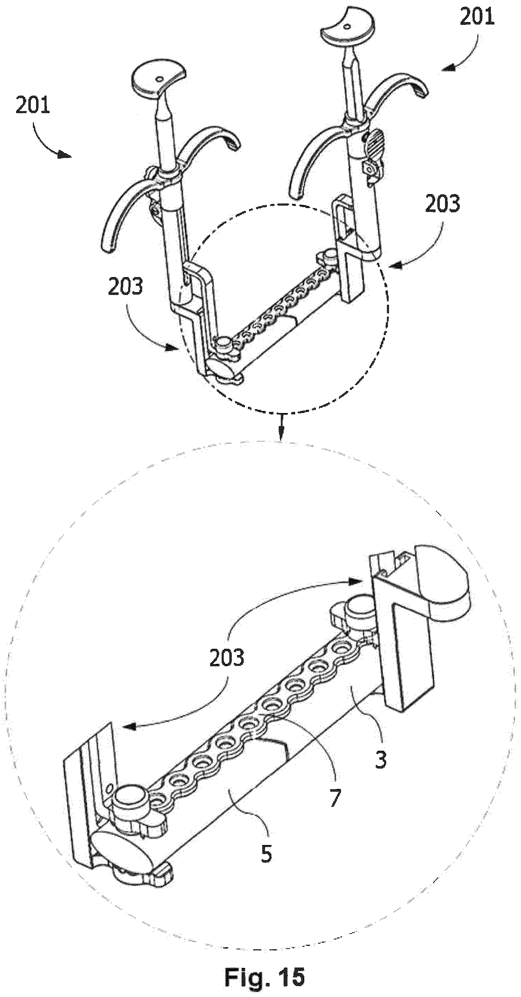

Fig. 15 shows a perspective view of two of the plate holding forceps 201 of Fig. 14 and an enlargement of said view, wherein each plate holding forceps 201 press the same bone plate 7 at opposite ends thereof to a different one of the two bone pieces 3, 5. Having two plate holding forceps 201 at the same time for use in the shown manner can help avoid a fracture dislocation, since each of the two bone pieces 3, 5 is pressed against the same rigid bone plate 7.

Accordingly, a plate holding forceps system may be provided, which comprises at least one, e.g. two, plate holding forceps 201. Alternatively, the plate holding forceps system may comprise at least one plate holding forceps 201 and at least one plate, such as the bone plate 7 shown herein, configured to be clamped by the clamp portion 203 of the plate holding forceps 201.

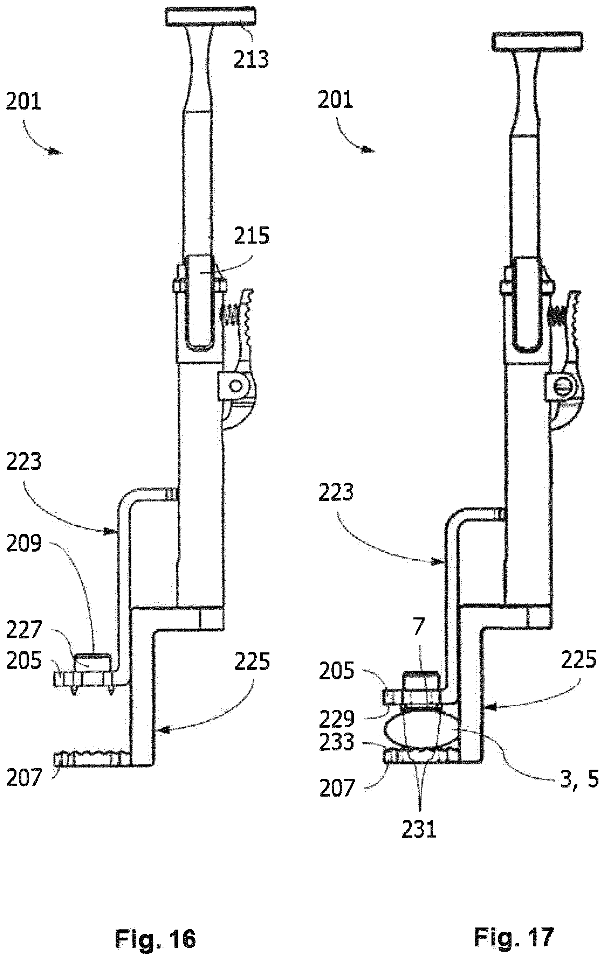

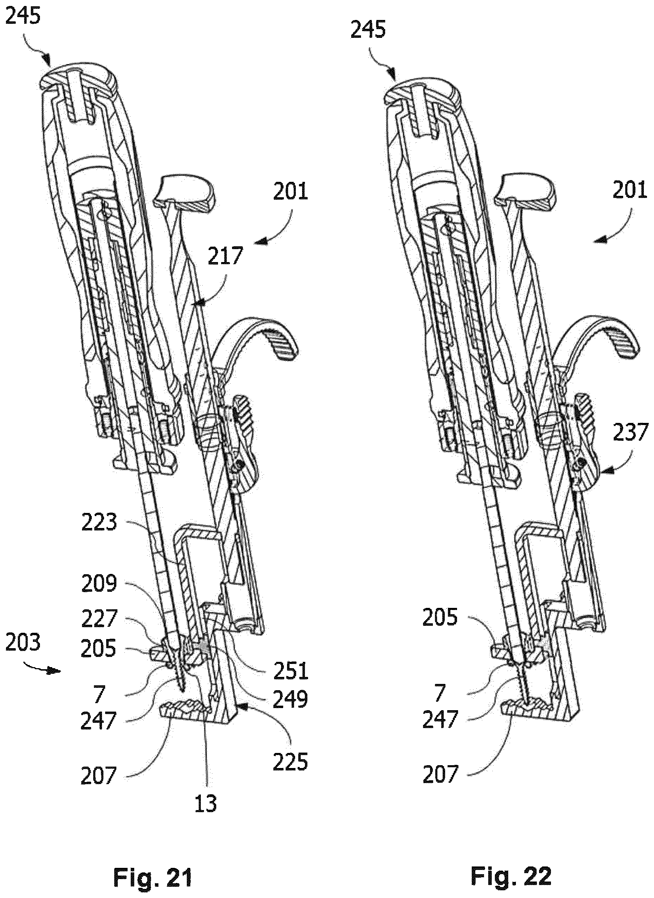

Fig. 16 shows a side view of the plate holding forceps 201 with the first jaw 205 and the second jaw 207 being distanced from each other. Here, the first jaw 205 is laterally spaced apart from the first handle part 213 and the second handle part 215 by a distance defined by a lateral extension of a first step portion 223. Correspondingly, the second jaw 207 is laterally spaced apart from the first handle part 213 and the second handle part 215 by a corresponding second step portion 225, wherein both step portions 223, 225 engage in a guiding manner, which is explained below with reference to Fig. 21.

The plate holding forceps 201 in this case further comprise a guiding protrusion 227, which protrudes from the first jaw 205 in a direction opposite to the second jaw 207.

The jaw through-bore 209 extends through the first jaw 205 and the guiding protrusion 227, which is illustrated in Fig. 21.

Fig. 17 shows a side view of the plate holding forceps 201 with the first jaw 205 and the second jaw 207 being distanced less from each other than in Fig. 16 and tightly holding the bone plate 7 to one of the two bone pieces 3, 5. The first jaw 205 comprises a pointed bottom surface 229 facing the second jaw 207. The pointed surface 229 is defined by at least two nubs 231, which extend in parallel to and distanced from each other orthogonally from the first jaw 205. In fact, the first jaw 205 can comprise a U-shaped cross-section, which is open towards the second jaw 207 and which comprises two nubs 231, for example, as the lateral walls of the U-shaped cross-section. The nubs 231 and/or the U-shaped cross-section are configured to laterally hold and/or fix the bone plate 7 in order to avoid a dislocation of the bone plate 7 relative to the first jaw 205.

The second jaw 207 comprises a corrugated top surface 233 facing the first jaw 205. The corrugated surface 233 is configured to better hold the bone piece 3, 5 in order to avoid a dislocation of the bone piece 3, 5 relative to the second jaw 207.

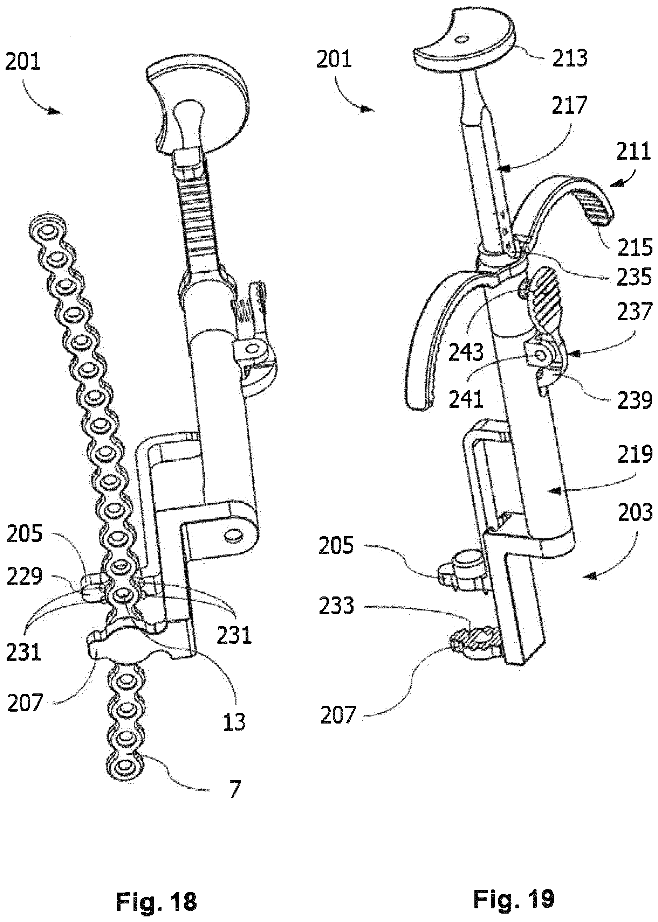

Fig. 18 shows a perspective view of the plate holding forceps 201 from the bottom side thereof with the bone plate 7 held by the first jaw 205. This view omits the bone piece between the bone plate 7 and the second jaw 207 to better illustrate the pointed surface 229 of the first jaw 205 with its four nubs 231. These nubs 231 radially enclose a portion of the bone plate 7, which portion structurally defines one of the openings 13. Thereby, the first jaw 205 is configured to automatically locate itself such that its jaw through bore 209 - see Fig. 14 - is coaxially aligned with an opening 13 of the bone plate 7.

Fig. 19 shows a perspective view of the plate holding forceps 201 from the top side thereof. The plate holding forceps 201 in this case further comprise a variable visual indication 235 indicating a parameter that depends on the actual distance between the first jaw 205 and the second jaw 207. Here, this visual indication 235 is provided by means of numbers engraved on the plunger part 217, such that their value decreases linearly in a direction away from the barrel part 219. When the plunger part 217 is moved into the barrel part 219, the first jaw 205 is moved closer to the second jaw 207 by an equal amount. The number on the plunger part 217, which is still completely visible and closest to the barrel part 219, indicates the screw length to be used for fastening the bone plate 7 to the bone piece 3, 5. The bone plate 7 and the bone piece 3, 5 being located between the first jaw 205 and the second jaw 207 will allow no further movement neither between the first jaw 205 and the second jaw 207 nor between the first handle part 213 and the second handle part 215.

The plate holding forceps 201 in this case further comprise a ratchet mechanism 237 having an active state, in which the jaws 205, 207 can be moved towards but not away from each other, and an inactive state, in which the jaws 205, 207 can be moved at least away from each other, i.e., towards and away from each other. Here, the ratchet mechanism 237 is located closer to the adjustment portion 211 than to the clamp portion 203 and configured to be manually operated with the same hand that operates the adjustment portion 211. For this purpose, the ratchet mechanism 237 comprises a manually operable switch 239 configured to move the ratchet mechanism 237 into the inactive state.

The ratchet mechanism 237 can be provided as shown in the figures. For example, the switch 239 may be provided as a rocker arm, which is pivotably hinged to the barrel part 219 to pivot around a barrel pivot axis 241 laterally fixed to the barrel part 219. One end of the switch 239 may be biased away from the barrel part 219 by means of a switch spring 243, such that the opposite other end of the switch 239 is forced against the plunger part 217 to assume the active state. This is achieved by a commonly known provision of a linear rack with teeth on the plunger part 217 and a pawl on the other end of the switch 239 capable of engaging the teeth - see Fig. 21 and Fig. 22 for an illustration. In order to move the switch 239 to its inactive state, the end of the switch 239 biased away from the barrel part 219 can be pushed towards the barrel part 219 against the biasing force of the switch spring 243, such that the opposite end of the switch 239 is pivoted away from the plunger part 219. Alternatively, the switch may be pivotably hinged to the first handle part 213 or the second handle part 215.

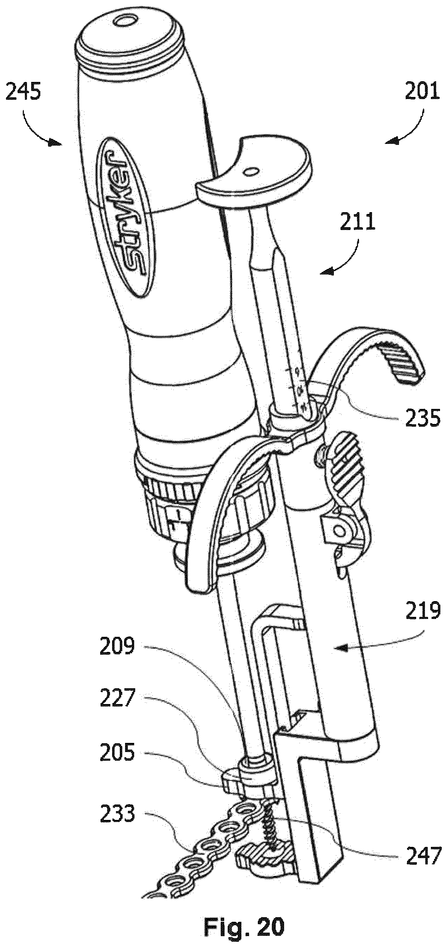

Fig. 20 shows a perspective view of the plate holding forceps 201 from the top side thereof with a screwdriver 245. The screwdriver 245 extends in parallel to the longitudinal extension direction of the adjustment portion 211 and its barrel part 219. A screw 247 corresponding in length to the visual indication 235 is inserted through the jaw through bore 209, i.e. through the guiding protrusion 227 and the first jaw 205, to fasten the bone plate 7 to the underlying bone piece, which is not shown here in order to better illustrate the screw 247.

Fig. 21 shows a cross-section of Fig. 20 in a state, where the screw 247 is not fully countersunk. Here, the jaw through-bore 209 comprises an inner funnel shape, which opens in a direction opposite to the second jaw 207, i.e. becomes narrower in a direction towards the second jaw 207. The funnel shaped jaw through-bore 209 ensures that the screw 247 will match the centre of the opening 13 of the bone plate 7 lying directly underneath the first jaw 205.

Further, it can be seen that one end of the first step portion 223 is attached to the plunger part 217. The other end of the first step portion 223 comprises a sliding member 249, which is slidably held within a rail slot 251 in the second step portion 225. As shown herein, the rail slot 251 may be a T-slot, i.e., a longitudinal groove with an undercut, and face the first step portion 223 to extend longitudinally in a clamping/unclamping direction of the clamp portion 203. This specific construction is one possibility to provide a movement of the jaws 205, 207 towards and away from each other along a straight line, and avoid a movement of the jaws 205, 207 in a plane orthogonal to this straight line.

Fig. 22 shows the cross-section of Fig. 21 in a state, where the screw 247 is fully countersunk. Once the screw 247 is countersunk through the bone plate 7 and into the bone piece, which is not show here, the screwdriver can be removed. Then, the ratchet mechanism 237 can be put into the inactive state to allow the jaws 205, 207 to be manually or automatically, e.g., by means of a spring biasing the jaws 205, 207 away from each other, distanced from each other. Finally, the plate holding forceps 201 can be removed entirely from the respective bone piece.

### List of Reference Signs

- 1 -: fractured bone
- 3, 5 -: bone pieces
- 7 -: bone plate
- 8 -: midline
- 9 -: top surface
- 11 -: bottom surface
- 13 -: openings
- 15 -: engagement part

- 101 -: plate bender
- 103 -: plate engagement portion
- 104 -: first axis
- 105 -: handle portion
- 107 -: pivoting mechanism
- 109 -: handle end
- 111 -: recess
- 113 -: mount
- 115 -: pivot axis
- 117 -: locking member
- 119 -: locking portions
- 121 -: pin
- 123 -: head
- 125 -: button
- 127 -: pin recess
- 129 -: locking spring
- 131 -: through-holes
- 133 -: intersections
- 135 -: main body
- 137 -: through-opening
- 139 -: bottom end
- 141 -: top end
- 143 -: displacement member
- 145 -: upper portion
- 147 -: lower portion
- 149 -: cylinder
- 151 -: flange
- 153 -: arms
- 155 -: engagement ends
- 157 -: engagement spring
- 159 -: engagement through-hole
- 161 -: drill

- 201 -: plate holding forceps
- 203 -: clamp portion
- 205 -: first jaw
- 207 -: second jaw
- 209 -: jaw through bore
- 211 -: adjustment portion
- 213 -: first handle part
- 215 -: second handle part
- 217 -: plunger part
- 219 -: barrel part
- 221 -: wings
- 223 -: first step portion
- 225 -: second step portion
- 227 -: guiding protrusion
- 229 -: bottom surface
- 231 -: nubs
- 233 -: top surface
- 235 -: visual indication
- 237 -: ratchet mechanism
- 239 -: switch
- 241 -: barrel pivot axis
- 243 -: switch spring
- 245 -: screwdriver
- 247 -: screw
- 249 -: sliding member
- 251 -: slot

## Claims

1. A plate bender (101) for bending a bone plate (7), the plate bender (101) comprising:
a plate engagement portion (103); and
a handle portion (105) coupled to the plate engagement portion (103);
wherein the handle portion (105) is coupled to the plate engagement portion (103) by a pivoting mechanism (107) configured to change an angular orientation between the handle portion (105) and the plate engagement portion (103);
wherein the plate engagement portion (103) comprises a displacement member (143) displaceable between a plate engagement position, in which a plate engagement is maintained, and a plate disengagement position, in which a plate disengagement is provided; and
wherein the plate engagement portion (103) has a longitudinal extension defining a first direction;
**characterized in that**
the handle portion (105) is pivotable around the plate engagement portion (103) in a plane angled to the first direction.

2. The plate bender (101) according to claim 1,
wherein the plane is orthogonal to the first direction.

3. The plate bender (101) according to claim 1 or 2,
wherein the plate bender (101) is configured maintain individual ones of a plurality of angular orientations between the handle portion (105) and the plate engagement portion (103).

4. The plate bender (101) according to claim 3,
wherein the angular orientations between the handle portion (105) and the plate engagement portion (103) are discrete.

5. The plate bender (101) according to claim 3 or 4,
comprising a locking member (117) displaceable between a locking position, in which a particular angular orientation is maintained, and an unlocking position, in which the handle portion (105) can pivot around the plate engagement portion (103) to change the angular orientation.

6. The plate bender (101) according to claim 5,
wherein the locking member (117) is configured to engage a particular one of multiple locking portions (119) to maintain a particular angular orientation, and
wherein the locking member (117) is provided at the plate engagement portion (103) and the locking portions (119) are provided on the handle portion (105), or vice versa.

7. The plate bender (101) according to claim 5 or 6,
wherein the locking member (117) is spring-biased to assume the locking position and configured to be manually displaced against the spring-bias to assume the unlocking position.

8. The plate bender (101) according to claim 7,
wherein the locking member (117) is configured to be manually pushed towards the handle portion (105) and thereby displaced to the unlocking position, and to return to the locking position when being released.

9. The plate bender (101) according to one of claims 5 to 8,
wherein the locking member (117) is configured to be manually pushed towards the handle portion (105) in a second direction, which is either identical or parallel to the first direction.

10. The plate bender (101) according to claim 9,
wherein the displacement member (143) is spring-biased to assume the plate engagement position and configured to be manually displaced against the spring-bias to the plate disengagement position.

11. The plate bender (101) according to claim 10,
wherein the displacement member (143) is configured to be manually pushed towards the plate engagement portion (103) to assume the plate disengagement position, and to return to the plate engagement position when being released.

12. The plate bender (101) according to one of claims 1 to 11,
wherein the plate engagement portion (103) comprises a through-hole (159) configured to act as a drill or screw guide.

13. A plate bender (101) system comprising:
a first and a second plate bender (101) according to one of claims 1 to 12 for left-hand and right-hand use, respectively.

14. A plate bender (101) system comprising:
at least one plate bender (101) as defined in claim 12; and
at least one bone plate (7) having a plate opening (13), wherein the plate engagement portion (103) is configured to align the plate opening (13), when the plate (7) is engaged, with the through-hole (159) configured to act as the drill or screw guide.

## Patentansprüche

1. Plattenbiegewerkzeug (101) zum Biegen einer Knochenplatte (7), wobei das Plattenbiegewerkzeug (101) umfasst:
einen Platteneingriffsabschnitt (103); und
einen Griffabschnitt (105), der mit dem Platteneingriffsabschnitt (103) gekoppelt ist;
wobei der Griffabschnitt (105) mit dem Platteneingriffsabschnitt (103) über einen Schwenkmechanismus (107) gekoppelt ist, der dazu konfiguriert ist, eine Winkelorientierung zwischen dem Griffabschnitt (105) und dem Platteneingriffsabschnitt (103) zu verändern;
wobei der Platteneingriffsabschnitt (103) ein Verstellelement (143) umfasst, das zwischen einer Platteneingriffsposition, in der ein Platteneingriff beibehalten wird, und einer Plattenausrückposition, in der eine Plattenausrückung vorgesehen wird, verstellbar ist; und
wobei der Platteneingriffsabschnitt (103) eine Längserstreckung aufweist, die eine erste Richtung definiert;
**dadurch gekennzeichnet, dass**
der Griffabschnitt (105) um den Platteneingriffsabschnitt (103) in einer zur ersten Richtung geneigten Ebene schwenkbar ist.

2. Plattenbiegewerkzeug (101) nach Anspruch 1,
wobei die Ebene orthogonal zur ersten Richtung ist.

3. Plattenbiegewerkzeug (101) nach Anspruch 1 oder 2,
wobei das Plattenbiegewerkzeug (101) dazu konfiguriert ist, einzelne von mehreren Winkelorientierungen zwischen dem Griffabschnitt (105) und dem Platteneingriffsabschnitt (103) beizubehalten.

4. Plattenbiegewerkzeug (101) nach Anspruch 3,
wobei die Winkelorientierungen zwischen dem Griffabschnitt (105) und dem Platteneingriffsabschnitt (103) diskret sind.

5. Plattenbiegewerkzeug (101) nach Anspruch 3 oder 4,
umfassend ein Verriegelungselement (117), das zwischen einer Verriegelungsposition, in der eine bestimmte Winkelorientierung beibehalten wird, und einer Entriegelungsposition, in der der Griffabschnitt (105) um den Platteneingriffsabschnitt (103) schwenkbar ist, um die Winkelorientierung zu ändern, verstellbar ist.

6. Plattenbiegewerkzeug (101) nach Anspruch 5,
wobei das Verriegelungselement (117) dazu konfiguriert ist, in einen bestimmten von mehreren Verriegelungsabschnitten (119) einzugreifen, um eine bestimmte Winkelorientierung beizubehalten, und
wobei das Verriegelungselement (117) am Platteneingriffsabschnitt (103) angeordnet ist und die Verriegelungsabschnitte (119) am Griffabschnitt (105) angeordnet sind, oder umgekehrt.

7. Plattenbiegewerkzeug (101) nach Anspruch 5 oder 6,
wobei das Verriegelungselement (117) federbelastet ist, um die Verriegelungsposition einzunehmen, und dazu konfiguriert ist, manuell gegen die Federkraft in die Entriegelungsposition bewegt zu werden.

8. Plattenbiegewerkzeug (101) nach Anspruch 7,
wobei das Verriegelungselement (117) dazu konfiguriert ist, manuell in Richtung des Griffabschnitts (105) gedrückt zu werden und dadurch in die Entriegelungsposition bewegt zu werden und bei Loslassen in die Verriegelungsposition zurückzukehren.

9. Plattenbiegewerkzeug (101) nach einem der Ansprüche 5 bis 8,
wobei das Verriegelungselement (117) dazu konfiguriert ist, manuell in einer zweiten Richtung in Richtung des Griffabschnitts (105) gedrückt zu werden, wobei die zweite Richtung entweder identisch mit der ersten Richtung oder parallel zur ersten Richtung ist.

10. Plattenbiegewerkzeug (101) nach Anspruch 9,
wobei das Verstellelement (143) federbelastet ist, um die Platteneingriffsposition einzunehmen, und dazu konfiguriert ist, manuell gegen die Federkraft in die Plattenausrückposition bewegt zu werden.

11. Plattenbiegewerkzeug (101) nach Anspruch 10,
wobei das Verstellelement (143) dazu konfiguriert ist, manuell in Richtung des Platteneingriffsabschnitts (103) gedrückt zu werden, um die Plattenausrückposition einzunehmen, und bei Loslassen in die Platteneingriffsposition zurückzukehren.

12. Plattenbiegewerkzeug (101) nach einem der Ansprüche 1 bis 11,
wobei der Platteneingriffsabschnitt (103) ein Durchgangsloch (159) umfasst, das dazu konfiguriert ist, als Bohr- oder Schraubenführung zu dienen.

13. Plattenbiegewerkzeug-System (101), umfassend:
ein erstes und ein zweites Plattenbiegewerkzeug (101) nach einem der Ansprüche 1 bis 12 für linkshändigen bzw. rechtshändigen Gebrauch.

14. Plattenbiegewerkzeug-System (101), umfassend:
zumindest ein Plattenbiegewerkzeug (101) nach Anspruch 12; und
zumindest eine Knochenplatte (7) mit einer Plattenöffnung (13), wobei der Platteneingriffsabschnitt (103) dazu konfiguriert ist, bei eingegriffener Platte (7) die Plattenöffnung (13) zu dem als Bohr- oder Schraubenführung dienenden Durchgangsloch (159) auszurichten.

## Revendications

1. Cintreuse pour plaque (101) destinée à cintrer une plaque vissée (7), la cintreuse pour plaque (101) comprenant:
une partie de mise en prise de plaque (103); et
une partie poignée (105) reliée à la partie de mise en prise de plaque (103);
dans lequel la partie poignée (105) est reliée à la partie de mise en prise de plaque (103) par un mécanisme de pivotement (107) conçu pour modifier l'orientation angulaire entre la partie poignée (105) et la partie de mise en prise de plaque (103);
dans lequel la partie de mise en prise de plaque (103) comprend un élément de déplacement (143) pouvant se déplacer entre une position de mise en prise de plaque, dans laquelle le mise en prise de la plaque est maintenue, et une position de libération de la plaque, dans laquelle la libération de la plaque est assurée; et
dans lequel la partie de mise en prise de plaque (103) présente une extension longitudinale définissant une première direction;
**caractérisée en ce que**
la partie poignée (105) peut pivoter autour de la partie de mise en prise de plaque (103) dans un plan formant un angle avec la première direction.

2. Cintreuse pour plaque (101) selon la revendication 1,
dans laquelle le plan est orthogonal à la première direction.

3. Cintreuse pour plaque (101) selon la revendication 1 ou 2,
dans lequel la cintreuse de plaque (101) est conçu pour maintenir chacune des différentes orientations angulaires possibles entre la partie poignée (105) et la partie de mise en prise de plaque (103).

4. Cintreuse pour plaque (101) selon la revendication 3,
dans lequel les orientations angulaires entre la partie poignée (105) et la partie de mise en prise de plaque (103) sont séparées.

5. Cintreuse pour plaque (101) selon la revendication 3 ou 4,
comprenant un élément de verrouillage (117) pouvant se déplacer entre une position de verrouillage, dans laquelle une orientation angulaire spécifique est maintenue, et une position de déverrouillage, dans laquelle la partie poignée (105) peut pivoter autour de la partie de mise en prise de plaque (103) afin de modifier l'orientation angulaire.

6. Cintreuse pour plaque (101) selon la revendication 5,
dans lequel l'élément de verrouillage (117) est conçu pour entrer en contact avec l'une des multiples parties de verrouillage (119) afin de maintenir une orientation angulaire spécifique, et
dans lequel l'élément de verrouillage (117) est disposé au niveau de la partie de mise en prise de plaque (103) et les parties de verrouillage (119) sont disposées sur la partie poignée (105), ou inversement.

7. Cintreuse pour plaque (101) selon la revendication 5 ou 6,
dans lequel l'élément de verrouillage (117) est sollicité par un ressort pour se placer en position de verrouillage et est conçu pour être déplacé manuellement à l'encontre de la force du ressort afin de se placer en position de déverrouillage.

8. Cintreuse pour plaque (101) selon la revendication 7,
dans lequel l'élément de verrouillage (117) est conçu pour être poussé manuellement en direction de la poignée (105) et être ainsi déplacé dans la position de déverrouillage, et pour revenir à la position de verrouillage lorsqu'on le relâche.

9. Cintreuse pour plaque (101) selon l'une quelconque des revendications 5 à 8,
dans lequel l'élément de verrouillage (117) est conçu pour être poussé manuellement en direction de la partie poignée (105) dans une deuxième direction, qui est soit identique, soit parallèle à la première direction.

10. Cintreuse pour plaque (101) selon la revendication 9,
dans lequel l'élément de déplacement (143) est sollicité par un ressort pour se placer en position de mise en prise avec la plaque et est conçu pour être déplacé manuellement, à l'encontre de la force du ressort, vers la position de libération de la plaque.

11. Cintreuse pour plaque (101) selon la revendication 10,
dans lequel l'élément de déplacement (143) est conçu pour être poussé manuellement dans la direction de la partie de mise en prise de plaque (103) afin de se placer en position de libération de plaque, et pour revenir en position de mise en prise de plaque lorsqu'on le relâche.

12. Cintreuse pour plaque (101) selon l'une quelconque des revendications 1 à 11,
dans laquelle la partie de mise en prise de plaque (103) comprend un trou traversant (159) conçu pour servir de guide pour le foret ou la vis.

13. Système de cintreuse pour plaque (101) comprenant:
une première et une deuxième cintreuse pour plaque (101) selon l'une quelconque des revendications 1 à 12, destinées respectivement à être utilisée par des gauchers et des droitiers.

14. Système de cintreuse pour plaque (101) comprenant:
au moins une cintreuse pour plaque (101) telle que définie dans la revendication 12; et
au moins une plaque vissée (7) comportant une ouverture (13), la partie de mise en prise de plaque (103) étant conçue pour aligner l'ouverture (13) de la plaque, lorsque la plaque (7) est en prise, le trou traversant (159) servant de guide pour le foret ou la vis.
